# EUROPEAN PATENT APPLICATION

(11) **EP 1 776 930 A1**
(43) Date of publication of application: **25.04.2007**
(21) Application number: 06121226.2
(22) Date of filing: 25.09.2006
(51) Int. Cl.: A61B 18/14

(54) **Device for endoscopic sphincterotomy**

(30) Priority: 18.10.2005 IT MI20051965
(71) Applicant: Innovamedica S.r.l., 20145 Milano (IT)
(72) Inventor: Cavalli, Marco, 20146, MILANO (IT)
(74) Representative: Tansini, Elio Fabrizio

(57) **Abstract**

A device for endoscopic sphincterotomy comprises a catheter (2) and a cutting wire (8) made of tungsten, installed close to a distal portion (7) of the catheter (2) and connectable to a source of electric energy.

## Description

The present invention relates to a device for endoscopic sphincterotomy, of the type comprising the features defined in the preamble of claim 1.

This device, commonly known as sphincterotome or papillotome, is used in the medical field for cannulation of the bile duct for ERCP (Endoscopic retrograde cholangiopancreatography) procedures or for carrying out the endoscopic sphincterotomy, i.e. for cutting the papilla of Vater, i.e. the point where the choledochus or common bile duct enters the duodenum, and the sphincteric muscles underlying the bile channels by means of a wire passed through by an electrodiathermic current, in order to be able to draw the gallstones out, for example.

It is known that the papillotome comprises a catheter of a highly smooth material, such as Teflon^{®}, inside which a steel wire runs which emerges some centimetres before reaching the distal end of the catheter and is fastened to the distal end itself. The wire is connected to a high-frequency generator and constitutes the electrosurgical knife cutting the papilla. In the closed or rest position, the distal portion of the catheter is straight and the wire lies parallel to and in contact with an outer portion of said catheter. Through pulling of the steel wire, the catheter at its distal length opens and takes an arc-shaped configuration in which the chord is represented by the wire. The endoscopic sphincterotomy is carried out by introducing the distal end of the papillotome still closed into the bile pathway and subsequently opening it to cut the sphincter of Oddi, from the inside of the common bile duct towards the duodenum lumen.

An important drawback connected with the endoscopic sphincterotomy carried out with the papillotomes of known type provided with a steel wire is represented by arising of premature complications taking place during the operation or immediately afterwards.

In particular, arising of acute pancreatitis in 0.5-2% of the cases has been noticed after sphincterotomy with papillotomes of known type.

In accordance with the Applicant's observations, arising of pancreatitis is mainly due to the thermal shock on the final length of the Wirsung's duct and on the surrounding pancreatic parenchima. In more detail, the Applicant has found that this phenomenon is to be ascribed to dissipation of the heat generated by the wire into the tissues surrounding the region of the cut that will cause an excessive enzymatic reaction with increase of the serum amylase beyond one thousand units.

In addition, the tissue edges cut with steel wires of known type appear to be serrated and this unevenness is the result of microlacerations, which means that also a mechanical shock has occurred.

In the light of the above, the present invention aims at providing a device for endoscopic sphincterotomy capable of solving the above mentioned drawbacks.

In particular, it is an aim of the present invention to propose a device for endoscopic sphincterotomy enabling the incidence of pancreatitis induced by the sphincterotomy operation to be reduced through reduction of the thermal shock as well as of the mechanical shock.

Another aim of the present invention is to conceive a device for endoscopic sphincterotomy enabling execution of the operation to be facilitated and accuracy of same to be increased, irrespective of the manual ability of the endoscopist.

The technical task mentioned and the aims specified are substantially achieved by a device for endoscopic sphincterotomy having the features recited in the appended claims.

Description of a preferred but not exclusive embodiment of a device for endoscopic sphincterotomy is now taken hereinafter by way of non-limiting example, and illustrated in the accompanying drawings, in which:
- Fig. 1 is a side view of a device for endoscopic sphincterotomy in accordance with the present invention;
- Fig. 2 shows a longitudinal cross-section view to an enlarged scale of a first portion of the device for endoscopic sphincterotomy seen in Fig. 1;
- Fig. 3 is a longitudinal cross-section view to an enlarged scale of a second portion of the device in Fig. 1; and
- Fig. 4 is a cross-sectional view of the device shown in Fig. 1.

With reference to the figures, a device for endoscopic sphincterotomy in accordance with the invention is generally denoted by reference numeral 1.

The device 1 comprises a catheter 2 provided at a proximal end 2a thereof, with a handgrip 3 and a hemostatic valve 4, both of known type and therefore not described in detail.

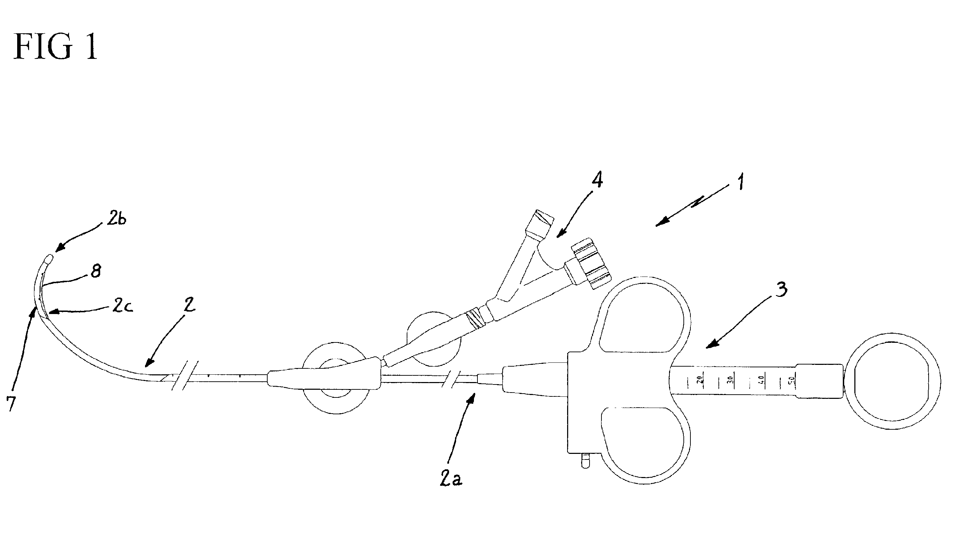

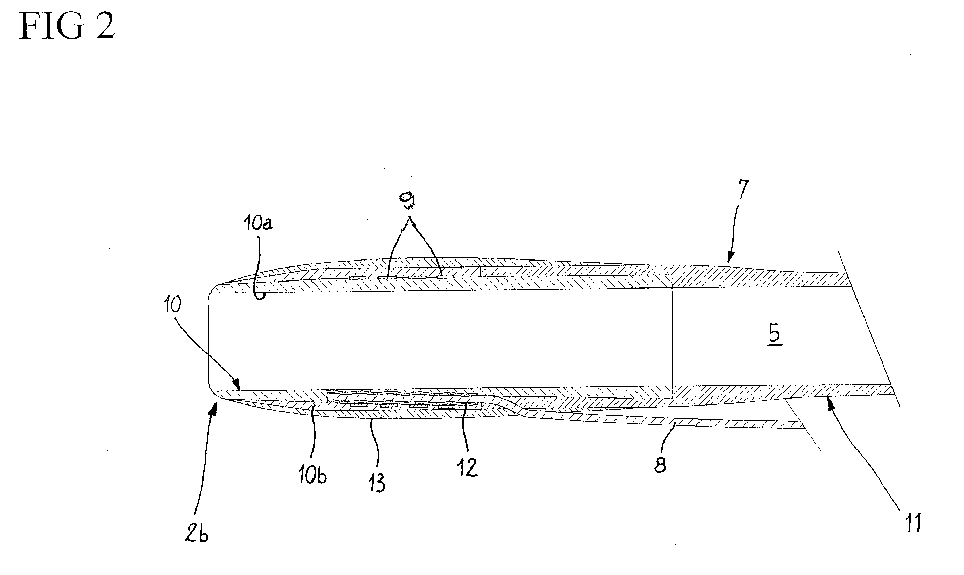

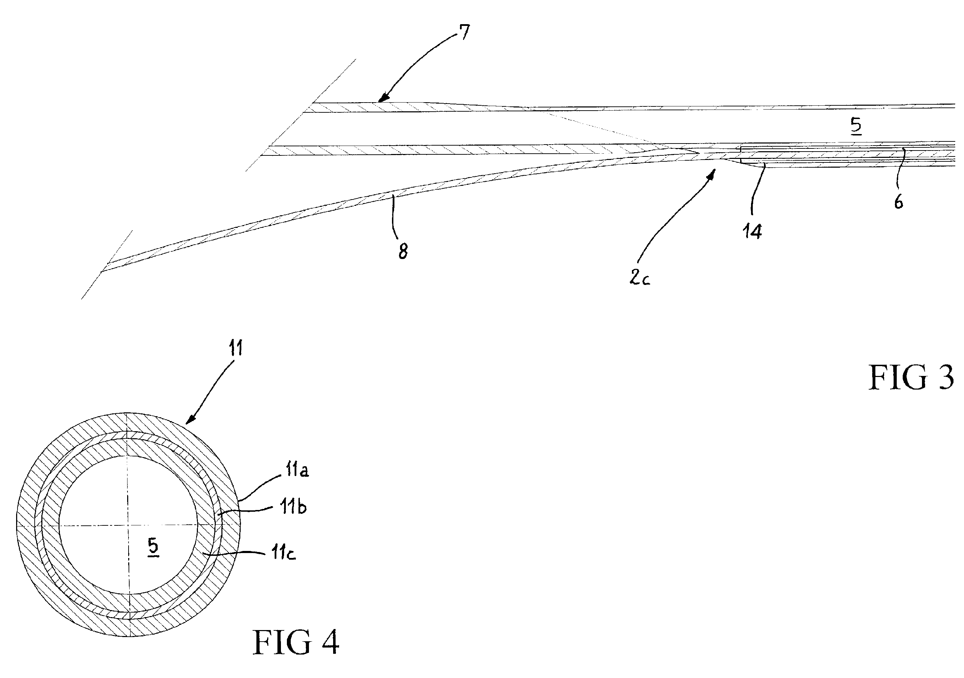

The catheter 2 has a main duct 5, shown in Figs. 2, 3 and 4, extending from the proximal end 2a over the whole length of the catheter 2 itself, until it opens close to a distal end 2b thereof, and an auxiliary duct 6 (partly shown in Fig. 3) extending from the proximal end 2a as far as a distal portion 7. The main duct 5 is used for introduction of probes and/or contrast liquids through the hemostatic valve 4. The auxiliary duct 6 houses a cutting wire 8 which is connected to and operated, in known manner, by the handgrip 3. The cutting wire 3 emerges from the catheter 2 at an outlet section 2c that is placed some centimetres back with respect to the distal end 2b and is fastened to the distal end 2b itself (Fig. 1 and 2).

The cutting wire 8 can be connected, in a manner known by itself, to a high-frequency generator, not shown, and constitutes the electrosurgical knife cutting the papilla. In the closed or rest position, the distal portion 7 of the catheter 2 delimited between the outlet section 2c and the distal end 2b, is straight and the cutting wire 8 lies parallel to and in contact with an outer portion of said catheter 2. By pulling the cutting wire 8 by means of the handgrip 3, the catheter 2 opens at its distal portion 7 and takes an arc-shaped configuration, the wire 8 constituting the chord of said arc (Fig. 1).

In the example shown, the sphincterotome 1 is of the type as conceived by Demling and Classen, but in any case, also falling within the scope of the present invention is the alternative embodiment not shown in which the papillotome is of the Sohma type. In the papillotome of the Sohma type the cutting wire is pushed instead of being pulled, takes the arc-shaped configuration and the catheter constitutes the chord of said arc.

Advantageously and unlike the papillotomes of known type, the cutting wire 8 of the device 1 in accordance with the present invention is made of tungsten. The tungsten wire 8 has a cross section included between 0.15 mm and 0.25 mm, and preferably equal to 0.20 mm. The tungsten wire 8 reaches higher temperatures than steel and less heat is dissipated in the tissues to be cut, the supply current being the same, so that a sharper cut is produced.

In addition, in order to avoid an excessive temperature increase of the distal end 2a of the catheter 2, a heat sink element 9 made of steel, preferably of AISI 304, and an insulation insert 10 made of polyether terketone, preferably prepared starting from the material known as VICTREX^{®} PEEK^{™} Polymer Naturale Granuli 151G, are inserted into the distal end 2a itself. The firm VICTREX^{®}is the only manufacturer of the semi-crystalline aromatic straight polymer VICTREX^{®} PEEK^{™}, the base unit of which is made up of oxy-1, 4-phenylene-oxy-1,4-phenylenene-carbonyl-1,4-phenylene.
The heat sink element 9 is placed in contact with the cutting wire 8 and its task is to dissipate the heat produced by wire 8, while the insulation insert 10 made of polyether terketone withstands much higher temperatures as compared with other types of plastic materials and is a heat insulator. In addition, due to its stiffness, insertion of the catheter 2 into the papilla is facilitated even in the absence of a guide wire usually employed in combination with the sphincterotome.

Referring particularly to the embodiment shown in Fig. 2, the insulation insert 10 comprises a reinforcing tube 10a which is partly inserted into the end of a distal tube 11 (seen in all figures) of the catheter 2. Spirally wound around the portion of the reinforcing tube 10a axially projecting from the distal tube 11 is the heat sink element 9. The distal end of the cutting wire 8 is inserted into a small tube made of steel 12 interposed and locked between the reinforcing tube 10a and the heat sink element 9. A second tube 10b is further mounted around the reinforcing tube 10a, at the distal end of the cutting wire 8 and of the heat sink element 9, said tube 10b being part of the insulation insert of polyether terketone 10 and lying in abutment against the distal tube 11.

An insert 11b of radiopaque material is preferably located in the distal portion 7, to enable easy identification of the position of the distal end 2b during the operation. To this end, as shown in the cross section of the distal tube 11 in Fig. 4, said distal tube 11 is of the multi-layer type and comprises three concentric layers. The outermost layer 11a and the innermost layer 11c delimiting the main duct 5, are made of a highly smooth material, such as polyamide, while the median layer 11b is made of a radiopaque material (barium sulphate) for 80%. Finally, the distal end 2b is wrapped in a highly smooth covering layer 13 such as polyamide, which contains barium sulphate too (Fig. 2).

A tube 14 of a shape memory material is inserted at the section of exit 2c of the cutting wire 8 from the catheter, the cutting wire 8 running in said tube (Fig. 3). "Shape memory material" means a material capable of recovering a pre-set macroscopic shape by effect of a change in the applied stress status. Preferably, the shape memory material used is a nickel and titanium alloy known under the name of NITINOL (acronym of NIckel TItanium Naval Ordinance Laboratory).

With reference to Fig. 3, the tube 14 of NITINOL is inserted at the end of the auxiliary duct 6 and when the papillotome 1 is in the rest position it takes a straight shape. By pulling the cutting wire 8 by means of the handgrip 3, the tube of NITINOL is urged by the wire 8 itself to take the pre-set arched shape, thus helping in deforming the whole distal portion 7 of the catheter 2 into an arc lying in a plane having a pre-established orientation relative to that of the handgrip 3 (Fig. 1) .

The invention achieves important advantages.

First of all, it has been noticed that adopting a wire 8 made of tungsten reduces the percentage of patients suffering from pancreatitis induced by the operation of sphincterotomy.

Without wishing to advance any binding theory, the Applicant's opinion is that the surprising results achieved by the invention are due to the fact that the tungsten wire allows heat dissipation in the tissues adjacent to the cut region to be reduced, which will reduce the thermal shock and enzymatic reaction.

In addition, the cut carried out with a tungsten wire appears to be sharper as compared with a cut carried out with the traditional wire made of steel and therefore also the mechanical shock on the cut region is reduced.

The heat generated by the wire is dissipated more quickly and more efficiently through the catheter also due to the fact of adopting inserts made of steel 9 and polyether terketone 10 that help in reducing the heat flow propagating into the tissues.

In addition, insertion of the catheter into the papilla even without a guide wire is facilitated due to the stiffness given by the insert of polyether terketone.

The shape memory tube 14 made of NITINOL prevents the wire from cutting the tube of the catheter made of plastic material during the operation and, since when submitted to deformation it does not flatten or get crushed, smooth running of the wire is ensured even when the catheter is so deformed that an angle of 90° is formed.

In addition, the shape memory tube 14 ensures that at the distal tube 11 the catheter 2 will always bend in a pre-established plane, because during insertion into the instrument it acts as a tiller for the rest of the catheter.

Finally, the radiopaque material positioned in the distal region 7 allows the endoscopist to identify the position and orientation of the cutting wire and to go on in a safer and more accurate manner in executing the operation.

## Claims

1. A device for endoscopic sphincterotomy comprising a catheter (2) and a cutting wire (8) installed close to a distal portion (7) of the catheter (2) and connectable to an electric energy source, **characterised in that** the cutting wire (8) is made of tungsten.

2. A device as claimed in claim 1, **characterised in that** the cutting wire (8) has a cross section of a diameter included between 0.15 mm and 0.25 mm, and preferably of 0.20 mm.

3. A device as claimed in claim 1, **characterised in that** a distal end (2b) of the catheter (2) comprises at least one reinforcing insert made of polyether terketone (10).

4. A device as claimed in claim 3, **characterised in that** the catheter (2) comprises a distal tube (11) and **in that** the reinforcing insert of polyether terketone (10) comprises a reinforcing tube (10a) fitted into the end of the distal tube (11).

5. A device as claimed in claim 1, **characterised in that** a distal end (2b) of the catheter (2) has a heat sink element (9), preferably made of steel, placed in contact with the cutting wire (8).

6. A device as claimed in claim 5, **characterised in that** the heat sink element (9) is coupled with an insulation insert (10), preferably made of polyether terketone.

7. A device as claimed in claim 5, wherein the catheter (2) comprises a distal tube (11), the insulation insert comprises a reinforcing tube (10a) fitted into the end of the distal tube (11) and the heat sink element (9) is a helical coil wound around the reinforcing tube (10a).

8. A device as claimed in claim 1, **characterised in that** it comprises a tube (14) made of shape memory material, located at a section (2c) of exit of the cutting wire (8) from the catheter and containing said cutting wire (8).

9. A device as claimed in claim 8, **characterised in that** the shape memory material is a nickel and titanium alloy.

10. A device as claimed in claim 1, **characterised in that** it comprises an insert (11c) of radiopaque material positioned in the distal portion (7).

11. A device as claimed in claim 10, **characterised in that** the catheter (2) comprises a multi-layer distal tube (11) and **in that** the insert (11b) of radiopaque material is defined by a median layer (11b) of said distal tube (11).
